# EUROPÄISCHE PATENTSCHRIFT

(11) **EP 0 695 607 B1**
(45) Veröffentlichungstag und Bekanntmachung des Hinweises auf die Patenterteilung: **24.03.1999**
(21) Anmeldenummer: 94810445.0
(22) Anmeldetag: 28.07.1994
(51) Int. Cl.: B27B 19/00, B23D 61/12, A61B 17/14

(54) **Sägeblatt für eine Knochensäge und Knochensäge mit einem solchen Sägeblatt**
Sawblade for bonesaw and bonesaw fitted with such a sawblade
Lame de scie à os et scie à os équipée d'une telle lame de scie

(43) Veröffentlichungstag der Anmeldung: 07.02.1996
(73) Patentinhaber: Sulzer Orthopädie AG, 6340 Baar (CH)
(72) Erfinder: Moser, Walter, Dr., CH-3126 Kaufdorf (CH); Kessler, Jean-Pierre, CH-3186 Düdingen (CH); Grunder, Beat, CH-3078 Richigen (CH)
(74) Vertreter: Trieblnig, Adolf

(56) Entgegenhaltungen:
- EP-A- 0 378 189
- EP-A- 0 382 483
- GB-A- 2 107 641
- US-A- 4 036 236

## Beschreibung

Die Erfindung betrifft ein Sägeblatt für eine Knochensäge entsprechend dem Oberbegriff des Patentanspruchs 1.

Sägeblätter der genannten Art, mit vorwiegend in Vorschubrichtung sich erstreckendem, länglichem Blattkörper, werden in der Regel zur Herstellung grossflächiger, durchgehender Sägeschnitte eingesetzt. Derartige Sägeblätter können mit geschränkten Sägezähnen oder, wie z.B. aus der Internationalen Patentanmeldung WO 94/05212 bekannt, mit nicht geschränkten Sägezähnen ausgeführt sein.

Ein Sägeblatt dergenannten Art ist aus der EP-A-0,378,189 bekannt. Das dort beschriebene Sägeblatt umfasst einen als biegesteifes Plattenteil ausgeführten Basiskörper, der an seinem freien Ende einen Fortsatz aufweist, der dünner als der Basiskörper ist. Am Ende des Fortsatzes sind die Sägezähne versetzt angeordnet vorgesehen. Die versetzt angeordneten Sägezähne weisen dabei eine Gesamthöhe auf, die etwa der Dicke des Basiskörpers entspricht oder die gerinfügig grösser ist.

Neben einer möglichst geringen Wärmeerzeugung zur Vermeidung von thermischer Schädigung des Knochengewebes sind insbesondere die Ebenheit und die Qualität der Schnittoberfläche von Bedeutung. Sägeblätter mit geschränkten Zähnen, durch welche ein Freiraum zur Vermeidung von zu grosser Reibung, d.h. Wärmeerzeugung, und für die Ableitung von Spangut gebildet wird, erzeugen zwar wenig Wärme im Sägespalt, führen jedoch zu einer rauhen Schnittoberfläche, wobei Beschädigungen im oberflächennahen Bereich des Knochens auftreten können. Hei Verwendung einer Sägeblattführung in Form einer Schlitzführung oder einer planen Auflage besteht zudem die Gefahr, dass durch Verschleiss der Führung, der Seitenflächen des Sägeblatts und insbesondere der über diese vorstehenden, geschränkten Sägezähne Metallabrieb in das Gewebe gelangt.

Das aus der Patentanmeldung WO 94/05212 bekannte Sägeblatt mit nicht geschränkten Sägezähnen kann direkt in einen Schlitz einer Sägeführung eingeführt werden. Dieses bekannte Sägeblatt ist mit einer kreisförmigen Durchtrittsöffnung versehen, welche sich über den grössten Teil der Breite des Blattkörpers erstreckt. Entsprechend wird der Querschnitt des Blattkörpers lokal geschwächt und damit die Steifigkeit des Blattkörpers lokal vermindert. Dünne, flexible Sägeblätter grösserer Länge können bei Verwendung einer Sägeblattführung im stets inhomogenen Knochen leicht abgelenkt werden, so dass wellige, nicht ausreichend passgenaue Sägeschnitte entstehen. Dicke Sägeblätter der bekannten Art ermöglichen zwar ebene grossflächige Sägeschnitte, verursachen jedoch wegen der grösseren Zahnbreite erhöhte Schnittkräfte und entsprechend mehr Wärme. Wegen des erhöhten Trägheitsmoments des Blattquerschnitts können stärkere Vibrationen auftreten, welche die Führung des Sägeblatts erschweren und den Motor der Antriebseinheit verstärkt belasten. Bei derartigen Ausführungen besteht zudem die Gefahr, dass im Bereich der an die ungeschränkten Sägezähne anschliessenden Seitenflächen des Sägeblatts Reibungswärme erzeugt und damit eine thermische Schädigung des Gewebes verursacht werden kann.

Der Erfindung liegt die Aufgabe zugrunde, ein verbessertes Sägeblatt zu schaffen, welches bei geringer Masse eine hohe Steifigkeit aufweist und welches die Herstellung grossflächiger, instrumentengeführter Sägeschnitte hoher Qualität bei geringer Wärmeerzeugung im Sägespalt ermöglicht. Zugleich soll eine vibrationsarme Führung des Blattkörpers und ein entsprechend vibrationsarmer Lauf des Motors der Antriebseinheit erzielt werden.

Diese Aufgabe wird erfindungsgemäss durch die kennzeichnenden Merkmale des Patentanspruchs 1 gelöst.

Das erfindungsgemäss ausgebildete Sägeblatt ermöglicht, aufgrund seiner durch die Gestaltung des Querschnitts des Blattkörpers optimierbaren, relativ hohen Steifigkeit, die Ausführung ebener Sägeschnitte, wobei durch die in Vorschubrichtung verlaufende nutenartige Vertiefung die mit dem Knochengewebe zusammenwirkende Kontaktfläche des Sägeblatts reduziert und zugleich ein Freiraum gebildet wird, durch den das Spangut aus dem Sägespalt abgeleitet und die wärmeerzeugende Reibung vermindert werden kann. Die durch die erfindungsgemässe Ausführung erzielbare hohe Qualität der Sägeschnitte bildet unter anderem eine wesentliche Voraussetzung für die erfolgreiche Implantation von Knieprothesen, insbesondere für die zementfreie Verankerung von Prothesenkomponenten, welche hohe Anforderungen an die Qualität der Knochenschnitte stellt.

Ausgestaltungen der Erfindung sind in den abhängigen Ansprüchen angegeben.

Eine Knochensäge mit einem derartigen Sägeblatt ist Gegenstand des Anspruchs 10.

Ein Verfahren zur Herstellung eines derartigen Sägeblatts ist Gegenstand des Anspruchs 11.

Weitere Einzelheiten ergeben sich aus der folgenden Beschreibung von in der Zeichnung schematisch dargestellten Ausführungsbeispielen der Erfindung, in Verbindung mit den Patentansprüchen. In der Zeichnung zeigen
- Fig.1: eine mit einem erfindungsgemäss ausgebildeten Sägeblatt bestückte Knochensäge in einer Seitenansicht,
- Fig.2: die Knochensäge in einer Teildraufsicht,
- Fig.3: das Sägeblatt nach Fig.1 in einer grösseren Darstellung,
- Fig.4: das Sägeblatt in einem Längsschnitt entsprechend der Linie IV - IV in Fig.3,
- Fig.5: einen Querschnitt des Sägeblatts entsprechend der Linie V - V in Fig.3,
- Fig.6: eine Einzelheit des Sägeblatts nach Fig.3 in einer grösseren Darstellung,
- Fig.7: einen Teilschnitt entsprechend der Linie VII - VII in Fig.6 und
- Fig.8: eine Einzelheit eines Sägeblatts nach einer abgewandelten Ausführungsform der Erfindung.

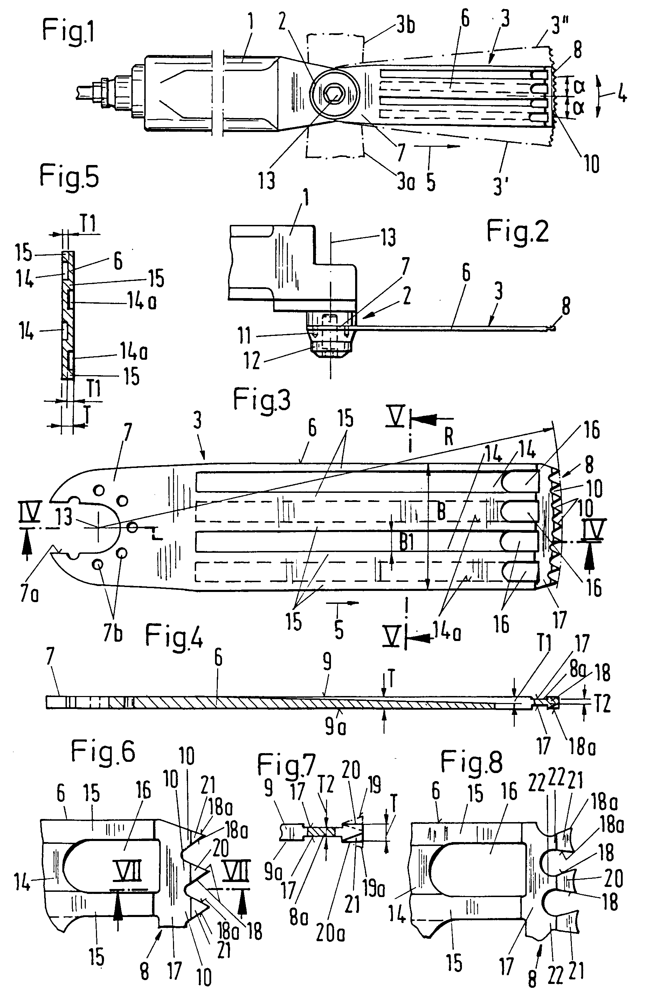

Die Knochensäge nach den Fig.1 und 2 enthält eine Antriebseinheit 1 mit einer Halterung 2 für ein Sägeblatt 3, welches entsprechend den Pfeilen 4 in oszillierenden Schnittbewegungen quer zu einer Vorschubrichtung (Pfeil 5) antreibbar ist. Das Sägeblatt 3 ist durch einen an der Halterung 2 in Vorschubrichtung (Pfeil 5) abstehend anbringbaren, länglichen Blattkörper 6 gebildet, der an einem Ende eine an der Halterung einspannbare Anschlusspartie 7 und an dem in Vorschubrichtung abstehenden anderen Ende eine Randpartie 8 mit an dieser ausgebildeten Sägezähnen 10 aufweist. Die Anschlusspartie 7 kann darstellungsgemäss mit einem in die Halterung 2 einführbaren Schlitz 7a und mit kreisförmig angeordneten Bohrungen 7b versehen sein, die zur Aufnahme von in der Halterung 2 angeordneten Führungsstiften 11 bestimmt sind. Entsprechend kann das Sägeblatt 3 in unterschiedlichen Winkelstellungen zwischen zwei strichpunktiert dargestellten Endstellungen 3a und 3b in der Halterung 2 positioniert und mittels einer Schraube 12 in der Halterung 2 eingespannt werden. Es sind zahlreiche, an sich bekannte Ausführungsvarianten zur dargestellten Anschlusspartie 7 möglich, deren Ausbildung nicht Gegenstand der vorliegenden Erfindung ist. Ueber die Halterung 2 kann das Sägeblatt 3 auf nicht weiter dargestellte Weise je um einen Winkel α um eine Schwenkachse 13 pendelnd zwischen zwei strichpunktiert angedeuteten Schwenkstellungen 3' und 3'' oszillierend angetrieben werden.

Der Blattkörper 6 ist mit einer Breitenabmessung B ausgeführt, die eine entsprechend den Schwenkstellungen 3' und 3'' begrenzte Schnittlänge definiert und die somit ein Einführen des Blattkörpers 6 in einen in Richtung der Schnittbewegungen (Pfeile 4) entsprechend begrenzten Arbeitsbereich gestattet.

Der Blattkörper 6 ist in Form eines biegesteifen Plattenteils mit einer definierten, darstellungsgemäss durch ebene Seitenflächen 9, 9a bestimmten grössten Dickenabmessung T ausgeführt, der auf beiden Seiten mit je zwei zwischen der Anschlusspartie 7 und der Randpartie 8 verlaufenden Vertiefungen in Form von Längsnuten 14 bzw. 14a versehen ist. Die Längsnuten 14 und 14a sind je durch zwei über ihre ganze Längserstreckung mit konstanter Dickenabmessung T durchlaufende Tragpartien 15 des Blattkörpers 6 begrenzt, wobei die auf der einen Seite angeordneten Längsnuten 14 je gegenüber den auf der anderen Seite angeordneten Längsnuten 14a versetzt sind. Entsprechend der Darstellung nach Fig.5 weist daher der Blattkörper 6 im Bereich der Längsnuten 14, 14a einen annähernd wellenartigen Querschnitt auf. Die Längsnuten 14, 14a sind je mit einem gegen die Anschlusspartie 7 hin abnehmenden Querschnitt, darstellungsgemäss mit abnehmender Tiefe T1 bei konstanter Breite B1, ausgeführt. Entsprechende Längsnuten können auch je mit gegen die Anschlusspartie 7 abnehmender Breite B1 - bei konstanter oder abnehmender Tiefe T1 - oder mit über ihre Länge konstanten Querschnittsabmessungen ausgeführt sein.

In einem an die Randpartie 8 anschliessenden Längenabschnitt des Blattkörpers 6 münden die Längsnuten 14 und 14a je in eine den Blattkörper 6 durchsetzende Durchtrittsöffnung 16. In der Randpartie 8 sind ferner zwei je auf einer Seite des Blattkörpers 6 angeordnete Vertiefungen in Form von Quernuten 17 ausgebildet, die sich in Richtung der Schnittbewegung (Pfeile 4) über die ganze Breite B des Blattkörpers 6 erstrecken.

Wie insbesondere aus der Fig.4 hervorgeht, können die im Blattkörper 6 gegeneinander versetzten Längsnuten 14 und 14a je mit einer Tiefe T1 ausgeführt sein, welche im Bereich der Durchtrittsöffnung 16 etwa der halben Dickenabmessung T des Blattkörpers 6 entspricht und welche darstellungsgemäss gegen die Anschlusspartie 7 hin kontinuierlich abnimmt. Es versteht sich, dass entsprechende Längsnuten 14 und 14a auch je mit einem von der dargestellten Ausführung abweichenden, z.B. trapezförmigen Querschnitt und/oder mit anderen Abmessungen, etwa mit einer zumindest über einen Teil ihrer Längserstreckung konstanten Tiefe T1 oder mit in einem Endabschnitt ihrer Längserstreckung gegen die Anschlusspartie 7 hin abnehmender Tiefe, ausgeführt sein können. Entsprechend der Darstellung nach den Fig.4 und 7 können die Quernuten 17 je mit einer solchen Tiefe ausgeführt sein, dass ein durch sie begrenzter Abschnitt 8a der Randpartie 8 eine Dickenabmessung T2 aufweist, die etwa der halben Dickenabmessung T des Blattkörpers 6 entspricht.

Es sind verschiedene abgewandelte Ausführungsformen des Blattkörpers 6 möglich, z.B. kann dieser mit einer grösseren oder kleineren als der dargestellten Breitenabmessung B ausgeführt und mit mehr oder weniger als vier Längsnuten 14 und 14a und einer entsprechenden Anzahl Durchtrittsöffnungen 16, z.B. auch mit einer einzigen Längsnut 14 und einer entsprechenden Durchtrittsöffnung 16, versehen sein.

Die Längsnuten 14 und 14a sowie die Quernuten 17 können je mittels eines Schleifwerkzeugs, z.B. einer Schleifscheibe, hergestellt werden. Wie insbesondere aus den Fig.4 und 7 hervorgeht, sind die Quernuten 17 je durch die Endabschnitte der Tragpartien 15 und einen die Sägezähne 10 enthaltenden Endabschnitt der Randpartie 8 begrenzt. Die Sägezähne 10 können je mit Schneidkanten 18 bzw. 18a ausgeführt sein, die gegeneinander je um ein Mass versetzt sind, welches der grössten Dickenabmessung T des Blattkörpers 6 entspricht. Dabei sind die Schneidkanten 18 und 18a je durch eine Aussenfläche 20 bzw. 20a begrenzt, die in einer durch die betreffende Seitenfläche 9 bzw. 9a des Blattkörpers 6 bestimmten Ebene liegt. Wie insbesondere aus der Fig.3 hervorgeht, können die Sägezähne 10 innerhalb eines durch ihre Spitzen bestimmten Kreisbogens mit einem Radius R angeordnet sein, dessen Mittelpunkt in der Anschlusspartie 7, im Bereich der Schwenkachse 13 des um diese pendelnd antreibbaren Blattkörpers 6 liegt.

Zur Herstellung der Schneidkanten 18 und 18a an den am Blattkörper 6 ausgebildeten, beim Beispiel nach den Fig.3 und 6 dreieckförmigen Sägezähnen 10 können diese je um ein einem Teil der Dickenabmessung T des Blattkörpers 6 entsprechendes Mass gegeneinander verschränkt werden, wobei die ursprünglich je in einer Ebene mit der Seitenfläche 9 bzw. 9a des Blattkörpers 6 liegenden Seitenwände 19 bzw. 19a der Sägezähne 10 - wie in Fig.7 strichpunktiert angedeutet - über die betreffende Ebene vorstehen. Diese vorstehenden Partien der Sägezähne 10 werden hierauf soweit abgeschliffen, dass ihre Aussenflächen 20 bzw. 20a je in der Ebene der betreffenden Seitenfläche 9 bzw. 9a des Blattkörpers 6 liegen und mit der beim Verschränken entsprechend verformten anderen Seitenwand 21 des betreffenden Sägezahns 10 konisch sich verjüngende Flanken begrenzen, mit denen sie die Schneidkanten 18 und 18a bilden. Auf diese Weise wird erreicht, dass alle Schneidkanten 18 und 18a innerhalb eines der Dickenabmessung T des Blattkörpers 6 entsprechenden, eng begrenzten Schnittbereichs angeordnet sind, so dass entsprechend schmale, unter grösstmöglicher Schonung des Knochengewebes ausführbare, präzise anlegbare Sägeschnitte mit relativ glatten Schnittflächen hergestellt werden können. Der Blattkörper 6 ist insbesondere auch zum Zusammenwirken mit einer nicht dargestellten Führungseinrichtung geeignet, welche einen Führungsschlitz aufweisen kann, der eine genaue und schonende Führung des Sägeblatts 3 gestattet. Durch die beschriebene Ausführung des Blattkörpers 6 mit nach der Schränkung plangeschliffenen Sägezähnen 10 kann eine Beschädigung der Schneidkanten 18, 18a und/oder der Führungseinrichtung sicher vermieden und damit die Gefahr der Entstehung von Metallabrieb vermindert werden.

Durch die im Blattkörper 6 ausgebildeten Längsnuten 14 und 14a ist ein massearmes Sägeblatt 3 erzielbar. Beim dargestellten Blattkörper 6, der z.B. mit einer Dickenabmessung T = ca. 0,8 - 1 mm ausgeführt sein kann, kann durch die Tragpartien 15 auch bei einer relativ grossen Blattlänge von z.B. 80 bis 120 mm oder mehr eine relativ hohe Steifigkeit des Blattkörpers 6 gewährleistet werden. Insbesondere bei Ausführungen mit von der Anschlusspartie 7 gegen die Randpartie 8 hin zunehmenden Tiefen T1 der Längsnuten 14 und 14a kann die Steifigkeit des Blattkörpers 6 an die beim Antrieb des Sägeblatts 3 auftretenden hohen Biegebeanspruchungen des Blattkörpers 6 angepasst werden.

Durch den oszillierenden Antrieb erfährt das Sägeblatt 3 in zunehmender Entfernung von der Schwenkachse 13 entsprechend zunehmende Beschleunigungen. Durch die Ausführung mit im Bereich der Randpartie 8 reduziertem bzw. mit zunehmender Entfernung von der Einspannstelle gegen diese Randpartie 8 hin abnehmendem Querschnitt des um die Schwenkachse 13 pendelnd antreibbaren Blattkörpers 6 sowie durch die an den Randbereich 8 unmittelbar anschliessenden Durchtrittsöffnungen 16 wird in diesem hoch beschleunigten Endabschnitt das Trägheitsmoment des Querschnitts des Blattkörpers 6 vermindert, während der mit zunehmender Nähe zur Einspannstelle zunehmend biegebelastete Längenabschnitt des Blattkörpers 6 eine entsprechend zunehmende Steifigkeit aufweist. Das Sägeblatt 3 ist somit dort steif, wo die grösste Biegebelastung auftritt und dort leicht, wo hohe Beschleunigungen auftreten. Der in der beschriebenen Weise ausgeführte Blattkörper 3 gestattet - auch bei einem Antrieb mit einer hohen Frequenz von z.B. mehr als 10'000 Hüben pro Minute - eine vibrationsarme Führung des Sägeblatts 3, wobei zugleich ein entsprechend vibrationsarmer Lauf des Motors der Antriebseinheit 1 gewährleistet werden kann.

Durch die Durchtrittsöffnungen 16 und die an diese anschliessenden Längsnuten 14 und 14a wird innerhalb des Grundquerschnitts des Bandkörpers 6, in unmittelbarer Nähe des Schnittbereichs, ein relativ grosses Auffangvolumen zur Aufnahme und zum Ableiten des bei den Schnittbewegungen in Form von Knochenmehl anfallenden Spanflusses gebildet, so dass vom Spanfluss weitgehend unbeeinträchtigte Schnittbewegungen ermöglicht werden.

Durch die entlang den Sägezähnen 10 bzw. 22 verlaufenden Quernuten 17 ist eine Freistellung der die Sägezähne 10 bzw. 22 enthaltenden Randpartie 8 und eine verbesserte Aufnahme des Knochenmehls unmittelbar hinter den Schneidkanten 18 und 18a sowie eine weitere Verbesserung der Ableitung des Spanflusses aus dem Schnittbereich erzielbar. Ein weiterer Vorteil dieser Ausführung besteht darin, dass die freigestellten Sägezähne 10 bzw. 22 das Knochengewebe im Schnittspalt lediglich mit ihren relativ kleinen Aussenflächen 20 und 20a, und damit jeweils auf einer im wesentlichen linienförmigen Bahn, mit entsprechend geringer Reibung berühren, so dass eine übermässige Erwärmung des Schnittbereichs verhindert und damit die Gefahr der Entstehung von Hitzenekrosen vermieden werden kann.

Gemäss Fig.8 kann ein entsprechender Blattkörper 6 mit Sägezähnen 22 in Form von annähernd trapezförmigen Doppelzähnen versehen sein, deren Ausbildung im übrigen derjenigen der Sägezähne 10 entspricht. Es versteht sich, dass auch Ausführungen mit beliebig anders geformten Sägezähnen möglich sind.

Nach einer Ausführungsform der Erfindung kann der Blattkörper 6 mit einer nicht dargestellten Schutzschicht aus einem abriebfesten, reibungsvermindernden Material, z.B. Kohlenstoff, versehen sein, so dass die Reibung im Schnittspalt und gegenüber der Sägeführung weiter verringert, die Verschleisseigenschaften des Blattkörpers 6 entsprechend verbessert und damit zugleich eine wesentlich erhöhte Standzeit des Sägeblatts 3 erzielt werden kann.

Ein relativ einfaches, kostengünstig durchführbares Verfahren zur Herstellung eines erfindungsgemäss ausgebildeten Sägeblatts 3 umfasst die folgenden Verfahrensschritte:
a) Aus einem biegesteifen Plattenteil, z.B. einem Stahlblech mit einer Dickenabmessung T = ca. 0,8 bis 1 mm, wird ein Blattkörper 6 gefertigt, an dem an einem Ende eine mit einer Antriebseinheit 1 kuppelbare Anschlusspartie 7, und an einer der Anschlusspartie 7 abgewandten Randpartie 8 eine Anzahl Sägezähne 10 bzw. 22 ausgebildet werden;
b) in den Blattkörper 6 wird mindestens eine zwischen der Anschlusspartie 7 und der Randpartie 8 verlaufende Vertiefung in Form einer Längsnut 14 bzw. 14a eingearbeitet;
c) in einem an die Randpartie 8 anschliessenden Endabschnitt der Längsnut 14 bzw. 14a wird mindestens eine den Blattkörper 6 durchsetzende Durchtrittsöffnung 16 ausgebildet, die zugleich als Werkzeugauslauf für das die Längsnut 14 bzw. 14a erzeugende Werkzeug, z.B. eine Schleifscheibe, dienen kann;
d) in der Randpartie 8 wird mindestens eine entlang den Sägezähnen 10 bzw. 22 verlaufende, zusätzliche Vertiefung in Form einer Quernut 17 hergestellt;
e) an den Sägezähnen 10 bzw. 22 werden scharf geschliffene Flanken ausgebildet.

Nach einer Ausführungsform können in einem weiteren Verfahrensschritt die Sägezähne 10 bzw. 22 gegeneinander verschränkt werden; in einem folgenden Verfahrensschritt kann der Blattkörper 6 einer Schleifvorrichtung zugeführt werden, durch welche die über die Ebene der jeweiligen Seitenfläche 9 bzw. 9a vorstehenden Partien 19 bzw. 19a der verschränkten Sägezähne 10, 22 je bis auf eine Aussenfläche 20 bzw. 20a plangeschliffen werden, die in einer durch die betreffende Seitenfläche 9 bzw. 9a bestimmten Ebene liegt.

In einem weiteren Verfahrensschritt kann die die Sägezähne 10 bzw. 22 enthaltende Randpartie 8 mit einer Aussenkontur in Form eines Kreisbogens ausgeführt werden, dessen Mittelpunkt im Bereich einer die Anschlusspartie 7 durchsetzenden Schwenkachse 13 liegt.

In einem weiteren Verfahrensschritt kann der Blattkörper 6 einer Beschichtungsvorrichtung zugeführt werden, durch welche zumindest auf die die Längsnuten 14 und 14a und Quernuten 17 begrenzenden Partien der Seitenflächen 9 und 9a je mit einer Schicht aus einem abriebfesten, reibungsvermindernden Material aufgebracht wird, welches z.B. Kohlenstoff enthält.

Das erfindungsgemäss ausgebildete Sägeblatt 3 ist, ausser für Operationen im Kniebereich, auch für beliebig andere Anwendungen, z.B. im Bereich des Hüftgelenks, geeignet.

## Patentansprüche

1. Sägeblatt für eine Knochensäge, mit einem über eine Antriebseinheit (1) quer zu einer Vorschubrichtung (5) oszillierend antreibbaren, an der Antriebseinheit (1) im wesentlichen in Vorschubrichtung (5) abstehend anbringbaren Blattkörper (6), der in Form eines biegesteifen Plattenteils ausgeführt ist und eine mit der Antriebseinheit (1) kuppelbare Anschlusspartie (7) aufweist, und der an einer der Anschlusspartie (7) in Vorschubrichtung (5) abgewandten Randpartie (8) mit Sägezähnen (10,22) versehen ist,
dadurch gekennzeichnet, dass der Blattkörper (6) auf mindestens einer Seite mit mindestens einer in Vorschubrichtung (5) zwischen der Anschlusspartie (7) und der die Sägezähne (10,22) enthaltenden Randpartie (8) verlaufenden, sich länglich zwischen diesen Partien erstreckenden, nutenartigen Vertiefung (14,14a) versehen ist, welche seitlich durch zwei über ihre Längserstreckung durchlaufende, die Anschlusspartie (7) und die Randpartie (8) verbindende Tragpartien (15) des Blattkörpers (6) begrenzt ist.

2. Sägeblatt nach Anspruch 1, dadurch gekennzeichnet, dass die Vertiefung (14,14a) zumindest in einem an die Anschlusspartie (7) angrenzenden Endabschnitt ihrer Längserstreckung mit einem gegen die Anschlusspartie (7) hin abnehmenden Querschnitt ausgeführt ist.

3. Sägeblatt nach Anspruch 1 oder 2, mit einer den Blattkörper (6) durchsetzenden Durchtrittsöffnung (16), dadurch gekennzeichnet, dass die Durchtrittsöffnung (16) in einem an die Randpartie (8) anschliessenden Längenabschnitt des Blattkörpers (6) ausgebildet ist.

4. Sägeblatt nach einem der vorangehenden Ansprüche, dadurch gekennzeichnet, dass bei mindestens zwei in Vorschubrichtung (5) verlaufenden Vertiefungen (14, 14a) mindestens eine erste Vertiefung (14) auf der einen Seite des Blattkörpers (6), und mindestens eine gegenüber dieser ersten Vertiefung (14) versetzte zweite Vertiefung (14a) auf der anderen Seite des Blattkörpers (6) angeordnet ist.

5. Sägeblatt nach einem der vorangehenden Ansprüche, dadurch gekennzeichnet, dass der Blattkörper (6) auf mindestens einer Seite mit einer entlang der die Sägezähne (10,22) enthaltenden Randpartie (8) quer zur Vorschubrichtung (5) verlaufenden, zusätzlichen nutenartigen Vertiefung (17) ausgeführt ist.

6. Sägeblatt nach einem der vorangehenden Ansprüche, dadurch gekennzeichnet, dass die Sägezähne (10,22) mit Schneidkanten (18,18a) ausgeführt sind, die je im wesentlichen um ein der grössten Dickenabmessung (T) des Blattkörpers (6) entsprechendes Mass gegenüber der benachbarten Schneidkante (18a,18) versetzt sind.

7. Sägeblatt nach Anspruch 6, dadurch gekennzeichnet, dass die Schneidkanten (18,18a) durch um die Dickenabmessung (T) des Blattkörpers (6) gegeneinander versetzte Aussenflächen (20,20a) der jeweils einander benachbarten Sägezähne (10,22) begrenzt sind, die je in einer durch eine der beiden Seitenflächen (9,9a) des Blattkörpers (6) bestimmten Ebene liegen.

8. Sägeblatt nach einem der vorangehenden Ansprüche, dadurch gekennzeichnet, dass die Sägezähne (10,22) innerhalb eines über die Breite (B) des Blattkörpers (6) verlaufenden Kreisbogens angeordnet sind, dessen Mittelpunkt in der Anschlusspartie (7) des um eine entsprechende Schwenkachse (13) pendelnd antreibbaren Blattkörpers (6) liegt.

9. Sägeblatt nach einem der vorangehenden Ansprüche, dadurch gekennzeichnet, dass der Blattkörper (6) mit einer Schutzschicht aus einem abriebfesten, insbesondere reibungsvermindernden Material, z.B. Kohlenstoff, versehen ist.

10. Knochensäge mit einem Sägeblatt nach einem der Ansprüche 1 bis 9.

11. Verfahren zum Herstellen eines Sägeblatts für eine Knochensäge, dadurch gekennzeichnet,
dass aus einem biegesteifen Blechteil ein Blattkörper (6) gefertigt wird, an dem eine mit einer Antriebseinheit (1) kuppelbare Anschlusspartie (7) und eine der Anschlusspartie (7) abgewandte Randpartie (8) mit Sägezähnen (10,22) ausgebildet werden,
dass in den Blattkörper (6) mindestens eine zwischen der Anschlusspartie (7) und der Randpartie (8) verlaufende, sich länglich zwischen diesen Partien erstreckende, nutenartige Vertiefung (14,14a) eingearbeitet wird,
dass in einem an die Randpartie (8) anschliessenden Längenabschnitt des Blattkörpers (6) mindestens eine den Blattkörper (6) durchsetzende Durchtrittsöffnung (16) ausgebildet wird,
dass in den Blattkörper (6) mindestens eine entlang der Randpartie (8) verlaufende, zusätzliche nutenartige Vertiefung (17) eingearbeitet wird, und
dass die Sägezähne (10, 22) scharf geschliffen werden.

12. Verfahren nach Anspruch 11, dadurch gekennzeichnet, dass die Sägezähne (10,22) gegeneinander verschränkt werden, und dass der Blattkörper (6) mit einer Schleifeinrichtung zusammengeführt wird, durch welche die über die Seitenflächen (9,9a) des Blattkörpers (6) vorstehenden Partien (19,19a) der verschränkten Sägezähne (10,22) je bis auf eine durch die betreffende Seitenfläche (9,9a) bestimmte Ebene abgeschliffen werden.

13. Verfahren nach Anspruch 11 oder 12, dadurch gekennzeichnet, dass die die Sägezähne (10,22) enthaltende Randpartie (8) des Blattkörpers (6) mit einer Aussenkontur in Form eines Kreisbogens ausgeführt wird, dessen Mittelpunkt im Bereich der Anschlusspartie (7) liegt.

14. Verfahren nach einem der Ansprüche 11 bis 13, dadurch gekennzeichnet, dass der Blattkörper (6) einer Beschichtungsvorrichtung zugeführt wird, durch welche zumindest auf die Seitenflächen (9,9a) des Blattkörpers (6) je eine Schicht aus einem abriebfesten, reibungsvermindernden Material aufgebracht wird.

## Claims

1. Saw blade for a bone saw with a blade body (6) which can be oscillatingly driven via a drive unit (1) transverse to a direction of advance (5) and which can be mounted on the drive unit (1) projecting substantially in the direction of advance (5), the blade body being executed in the form of a plate member stiff in bending and having a connection portion (7) which can be coupled with the drive unit (1), and being provided with saw teeth (10, 22) at an edge portion (8) facing away from the connection portion (7) in the direction of advance (5), characterised in that the blade body (6) is provided on at least one side with at least one groove-like recess (14, 14a) which extends in the direction of advance (5) between the connection portion (7) and the edge portion (8) containing the saw teeth (10, 22) elongatedly extending between these portions, with the recess being bounded laterally by two carrier portions (15) of the blade body (6) which extend over the length of the groove and connect the connection portion (7) and the edge portion (8).

2. Saw blade in accordance with claim 1, characterised in that the recess (14, 14a) is formed with a cross-section which decreases towards the connection portion (7) at least in an end section of its longitudinal extent which adjoins the connection portion (7).

3. Saw blade in accordance with claim 1 or 2, with an opening (16) passing through the blade body (6), characterised in that the opening (16) is formed in a longitudinal section of the blade body (6) which adjoins the edge portion (8).

4. Saw blade in accordance with one of the preceding claims, characterised in that with at least two recesses (14, 14a) extending in the direction of advance (5), at least a first recess (14) is arranged on the one side of the blade body (6), and at least one second recess (14a) is arranged on the other side of the blade body (6) offset from this first recess (14).

5. Saw blade in accordance with one of the preceding claims, characterised in that the blade body (6) is formed on at least one side with an additional groove-like recess (17) which extends transverse to the direction of advance (5) along the edge portion (8) which contains the saw teeth (10, 22).

6. Saw blade in accordance with one of the preceding claims, characterised in that the saw teeth (10, 22) are formed with cutting edges (18 and 18a) which are each offset from the neighbouring cutting edge (18a or 18) substantially by an amount corresponding to the largest thickness dimension (T) of the blade body (6).

7. Saw blade in accordance with claim 6, characterised in that the cutting edges (18 and 18a) are bounded by the outer surfaces (20 or 20a) of the respectively adjacent saw teeth (10, 22), with the outer surfaces (20 or 20a) being mutually offset by the thickness dimension (T) of the blade body (6), and each lying in a plane determined by one of the two side surfaces (9 or 9a) of the blade body (6).

8. Saw blade in accordance with one of the preceding claims, characterised in that the saw teeth (10, 22) are arranged within a circular arc extending over the width (B) of the blade body (6), with the centre of the circular arc lying in the connection portion (7) of the blade body (6) which can be driven in a manner of a pendulum about a corresponding pivot axis (13).

9. Saw blade in accordance with one of the preceding claims, characterised in that the blade body (6) is provided with a protective layer comprising an abrasion-resistant material, in particular a friction-reducing material, for example carbon.

10. Bone saw with a saw blade in accordance with one of the claims 1 to 9.

11. Method for the manufacture of a saw blade for a bone saw, characterised in that
a blade body (6) is made from a sheet metal part which is stiff in bending, on which are formed a connection portion (7) which can be coupled with a drive unit (1), and an edge portion (8) with saw teeth (10, 22) which faces away from the connection portion (7),
in that at least one groove-like recess (14, 14a) extending between the connection portion (7) and the edge portion (8) is formed in the blade body (6) elongatedly extending between these portions,
in that at least one opening (16) which goes through the blade body (6) is formed in a longitudinal section of the blade body (6) adjoining the edge portion (8),
in that at least one additional groove-like recess (17) extending along the edge portion (8) is formed in the blade body (6), and
in that the saw teeth (10, 22) are sharply ground.

12. Method in accordance with claim 11, characterised in that the saw teeth (10, 22) are mutually staggered, and in that the blade body (6) is brought together with a grinding apparatus through which the portions (19 or 19a) of the staggered saw teeth (10, 22) which project beyond the side surfaces (9 and 9a) of the blade body (6) are each ground down to a plane determined by the corresponding side surface (9, 9a).

13. Method in accordance with claim 11 or 12, characterised in that the edge portion (8) of the blade body (6) containing the saw teeth (10, 22) is formed with an outer contour in the form of a circular arc, the centre of which lies in the region of the connection portion (7).

14. Method in accordance with one of the claims 11 to 13, characterised in that the blade body (6) is brought to a coating device through which a layer comprising a friction-reducing, abrasion-resistant material is applied, at least on each of the side surfaces (9, 9a) of the blade body (6).

## Revendications

1. Lame de scie pour une scie à os, avec un corps de lame (6) pouvant être entraîné d'une manière oscillante par une unité d'entraînement (1) transversalement à une direction d'avance (5), applicable à l'unité d'entraînement (1) essentiellement de manière saillante dans la direction d'avance (5), qui est réalisé sous la forme d'une partie de plaque rigide en flexion et qui présente une partie de raccordement (7) pouvant être accouplée à l'unité d'entraînement (1) et qui est pourvu à une partie de bord (8) détournée de la partie de raccordement (7) dans la direction d'avance (5) de dents de scie (10, 22), caractérisée en ce que le corps de lame (6) est pourvu sur au moins un côté d'au moins un creux en forme de rainure (14, 14a) s'étendant dans la direction d'avance (5) entre la partie de raccordement (7) et la partie de bord (8) contenant les dents de scie (10, 22), s'étendant en longueur entre ces parties, qui est délimité sur le côté par deux parties de support (15) du corps de lame (6) continues sur leurs extensions longitudinales, reliant la partie de raccordement (7) et la partie de bord (8).

2. Lame de scie selon la revendication 1, caractérisée en ce que le creux (14, 14a) est réalisé au moins dans un tronçon d'extrémité, avoisinant la partie de raccordement (7), de son extension longitudinale avec une section transversale diminuant vers la partie de raccordement (7).

3. Lame de scie selon la revendication 1 ou 2, avec une ouverture de passage (16) traversant le corps de lame (6), caractérisée en ce que l'ouverture de passage (16) est réalisée dans un tronçon longitudinal du corps de lame (6) faisant suite à la partie de bord (8).

4. Lame de scie selon l'une des revendications précédentes, caractérisée en ce que dans le cas d'au moins deux creux (14, 14a) s'étendant dans la direction d'avance (5), au moins un premier creux (14) est disposé sur un côté du corps de lame (6) et au moins un deuxième creux (14a) décalé par rapport à ce premier creux (14) est disposé sur l'autre côté du corps de lame (6).

5. Lame de scie selon l'une des revendications précédentes, caractérisée en ce que le corps de lame (6) est réalisé sur au moins un côté avec un creux additionnel (17) en forme de rainure s'étendant le long de la partie de bord (8) contenant les dents de scie (10, 22), transversalement à la direction d'avance (5).

6. Lame de scie selon l'une des revendications précédentes, caractérisée en ce que les dents de scie (10, 22) sont réalisées avec des arêtes de coupe (18, 18a) qui sont décalées chacune essentiellement d'une mesure correspondant à la plus grande dimension d'épaisseur (T) du corps de lame (6) par rapport à l'arête de coupe avoisinante (18a, 18).

7. Lame de scie selon la revendication 6, caractérisée en ce que les arêtes de coupe (18, 18a) sont délimitées par des surfaces extérieures (20, 20a) décalées l'une par rapport à l'autre selon la dimension d'épaisseur (T) du corps de lame (6) des dents de scie (10, 22) respectivement avoisinantes qui se situent chacune dans un plan défini par les deux surfaces latérales (9, 9a) du corps de lame (6).

8. Lame de scie selon l'une des revendications précédentes, caractérisée en ce que les dents de scie (10, 22) sont disposées à l'intérieur d'un arc de cercle s'étendant sur la largeur (B) du corps de lame (6) dont le centre se situe dans la partie de raccordement (7) du corps de lame (6) pouvant être entraîné d'une manière pendulaire autour d'un axe de pivotement correspondant (13).

9. Lame de scie selon l'une des revendications précédentes, caractérisée en ce que le corps de lame (6) est pourvu d'une couche de protection en un matériau résistant à l'usure, diminuant notamment le frottement, par exemple en carbone.

10. Scie à os comportant une lame de scie selon l'une des revendications 1 à 9.

11. Procédé de fabrication d'une lame de scie pour une scie à os, caractérisé
en ce qu'on fabrique à partir d'une partie en tôle résistant à la flexion un corps de lame (6) auquel sont réalisées une partie de raccordement (7) pouvant être accouplée à une unité d'entraînement (1) et une partie de bord (8) détournée de la partie de raccordement (7), avec des dents de scie (10, 22),
en ménageant dans le corps de lame (6) au moins un creux en forme de rainure (14, 14a) s'étendant entre la partie de raccordement (7) et la partie de bord (8), s'étendant en longueur entre ces parties,
en ce qu'il est réalisé dans un tronçon longitudinal du corps de lame (6) faisant suite à la partie de bord (8) au moins une ouverture de passage (16) traversant le corps de lame (6),
en ce qu'il est ménagé dans le corps de lame (6) au moins un creux additionnel en forme de rainure (17), s'étendant le long de la partie de bord (8) et
en ce que les dents de scie (10, 22) sont affûtées d'une manière tranchante.

12. Procédé selon la revendication 11, caractérisé en ce que les dents de scie (10, 22) sont croisées les unes contre les autres et en ce que le corps de lame (6) est réuni avec un dispositif de meulage par lequel les parties (19, 19a) des dents de scie croisées faisant saillie sur les surfaces latérales (9, 9a) du corps de lame (6) sont meulées chacune jusqu'à un plan défini par la surface latérale concernée (9, 9a).

13. Procédé selon la revendication 11 ou 12, caractérisé en ce que la partie de bord (8) du corps de lame (6) contenant les dents de scie (10, 22) est réalisée avec un contour extérieur sous la forme d'un arc de cercle dont le centre se situe au voisinage de la partie de raccordement (7).

14. Procédé selon l'une des revendications 11 à 13, caractérisé en ce que le corps de lame (6) est amené à un dispositif de revêtement par lequel il est appliqué au moins sur les surfaces latérales (9, 9a) du corps de lame (6) respectivement une couche en un matériau résistant à l'usure, diminuant le frottement.
